Europäisches Patentamt

European Patent Office

Office européen des brevets

(11) Publication number: **0 368 583**
**A1**

## EUROPEAN PATENT APPLICATION

(21) Application number: **89311451.2**

(22) Date of filing: **06.11.89**

(51) Int. Cl.5: **A61F 5/03**

(30) Priority: **05.11.88 GB 8825970**

(43) Date of publication of application:
**16.05.90 Bulletin 90/20**

(84) Designated Contracting States:
**AT BE CH DE ES FR GB IT LI NL SE**

(71) Applicant: **SETON PRODUCTS LIMITED**
**Tubiton House Medlock Street**
**Oldham Lancashire OL1 3HS(GB)**

(72) Inventor: **Norris, Alan Hedley**
**120 Saddle Mountain Road Rome**
**Georgia 30161(US)**

(74) Representative: **Funge, Harry et al**
**M'CAW & CO. 41-51 Royal Exchange Cross**
**Street**
**Manchester M2 7BD(GB)**

(54) **Post-operative support belt.**

(57) A post-operative support belt is proposed which is of a length to extend wholly about the torso of a patient, the overlapped ends of the belt being such as loosely to hold the belt in position about the patient when not required for use but being capable of being used by the patient in belt tension inducing manner to provide support in the event of coughing. Flaps (16) are provided at the respective belt ends, the said flaps each including an elongate slot (17) to facilitate gripping of the belt ends. Preferably the belt comprises two bands (11, 12) secured together in overlapped disposition by releasable fastening means (13), thereby to facilitate application of the belt to the patient.

Fig.1

## POST-OPERATIVE SUPPORT BELT

The invention concerns a post-operative support belt and has more particular reference to a device for the reduction of pain caused by coughing and the saving of life by prevention of post anesthetic pneumonia.

When patients have ingested anesthetics into the lungs during operations, it is essential that they cough up and expectorate the fluid caused by the anesthetic on the lungs during the 48 hours following the surgery. Failure to do so can cause pneumonia. However, the tensing of the muscles during a cough, causes excruciating pain and patients try not to cough to avoid the pain. They feel that they may tear out any stitches and cause internal damage. Thus any device which helps them to cough is of great benefit.

The object of the invention is to provide such a device.

According to the present invention there is proposed a post-operative support belt comprising a belt structure to encircle the patient's torso and of a length to provide overlapping free belt ends, and means to hold the belt structure loosely in endless form, as about the patient in the non-use condition thereof, the free belt ends being adapted and arranged to provide a facility for patient-induced and patient maintained tensioning of the belt about the torso through the said ends.

According to a preferred feature, the belt comprises band parts arranged in overlapping end-to-end disposition and connected together in such disposition by separable fastening means, the free ends of the band parts including spaced side-by-side tape-like elements and the tape-like elements of the respective ends being arranged in interdigitated disposition.

According to a further preferred feature, the free ends of the tape-like elements at the band ends are secured to respective apertured flaps.

The ideal support would be a very tightly wound belt which would support the appropriate part of the torso. However, the belt tension and internal pressure caused by such tension level which gave confidence to the patient would be deleterious if applied over long periods. Hence the belt, which should have sufficient tension to hold it in place, but no more, must be capable of being tensioned by the patient at those times when he feels he must cough. Since only the patient is aware of this, it follows that the belt must be tensioned by the patient.

Preferably the belt will be adjustable to patients of different chest and waist measurements and will be capable of quick removal and replacement, so the nurse may inspect the operational wound and dressings thereon. The belt will completely encircle the patient, so that all pressure is inwards and there are no sideways forces which could disturb stitches or dressings. Further, it has been found, by personal experience, that the tensing of all muscles on the front of the body is beneficial, and thus it is preferable that the belt be tensioned by crossing the arms in front of the body and tensing the belt by a pushing action which uses the pectoral muscles. It is to be understood, however, that the belt may be tensioned by uncrossed arms and a pulling motion.

The invention will now be described further, by way of example, by reference to the accompanying diagrammatic drawings illustrating several embodiments thereof and in which:-

Fig. 1 is a perspective view of a preferred embodiment;

Fig. 2 is a plan view of the arrangement shown in Fig. 1;

Fig. 3 shows an alternative form of grip position for the belt shown in Figs. 1 and 2;

Fig. 4 shows an alternative form of belt end to that shown in Fig. 1; and

Figs. 5a to c illustrate an alternative embodiment.

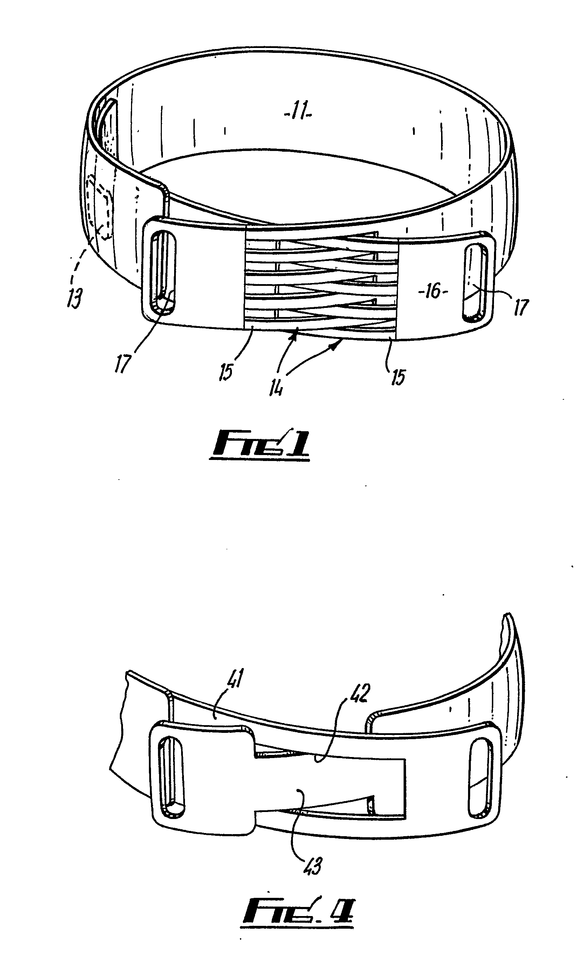

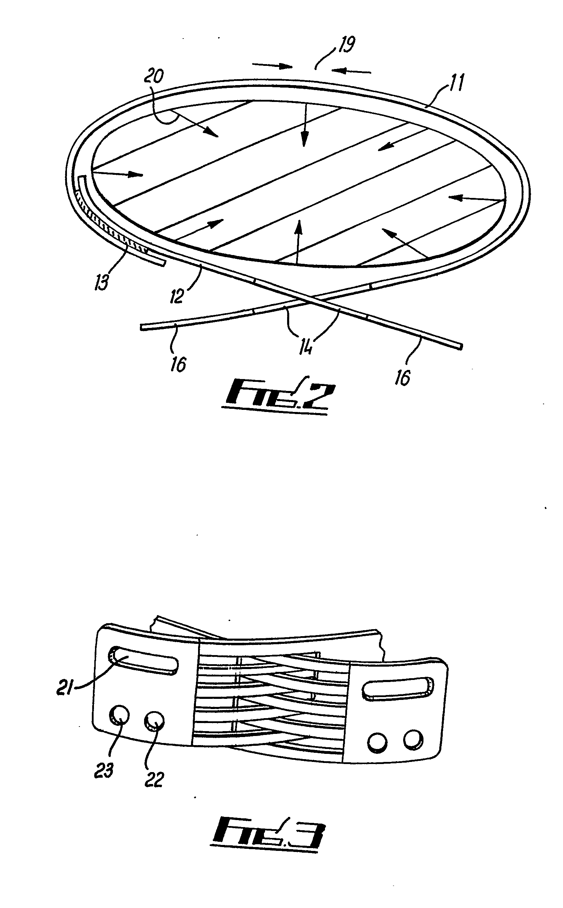

Referring now to the drawings, and particularly to Figs. 1 and 2 thereof, a post-operative support belt constructed in accordance with the invention comprises two bands 11, 12 arranged in end-to-end overlapping disposition and releasably secured together in the overlapping region by a hook and loop fastening means 13, fastening means of the kind sold under the Trade Mark VELCRO being found to be particularly suitable. The free ends of the respective bands 11, 12 are slitted to provide spaced, parallel tape-like elements 14, the elements 14 of the respective ends 11, 12 being interdigitated in the manner clearly shown in Fig. 1. The remote ends 15 of the tape-like elements at each band end are secured to respective end flaps 16, there being an elongate slot 17 in each flap extending in the transverse direction of the belt and defining respective grip portions thereon.

The bands 11, 12 will preferably be of woven construction, and will typically be 15 cms wide. The combined lengths of the bands will be such that the belt will comfortably encircle a persons torso, the fastening means 13, which means may be of appreciable longitudinal extent, providing a facility for adjustment according to the size of the patient.

Referring now to Fig. 2, the shaded portion 18 represents the torso of the patient and the belt is in position around the patient's waist. The arrows 19 indicate that the belt is under tension and the arrows 20 indicate the inwards support pressure resulting from such tension. As is apparent, the circumference of the belt can be reduced by pulling on flaps 16.

Thus a patient wearing the belt can cause increased radially inward pressure on his torso by pulling or pushing on flaps 16. The slitting of the ends of the bands and the interdigitation thereof represents merely a preferred way of allowing the reduction of belt circumference whilst preserving a complete wrap around the torso.

The belt may readily be fitted simply by wrapping the same around the patient and closing the fastening means. Wound inspection is readily achieved by pulling apart the VELCRO fastener and loosening the belt. Means other than VELCRO can be used such as, for example, laces. Hard objects such as buckles are preferably avoided as the patient may lie on them, causing discomfort. The width of the belt should be such that its principal pressure is between the ribs and the hip bones which would otherwise prevent inwards pressure if the belt was upon them. It may be advantageous to place a pad under the belt if it passes over the operation area.

As will be appreciated, support may be provided only at such times as it is needed, constriction of the abdomen or other parts of the torso at other times being avoided when it is not needed.

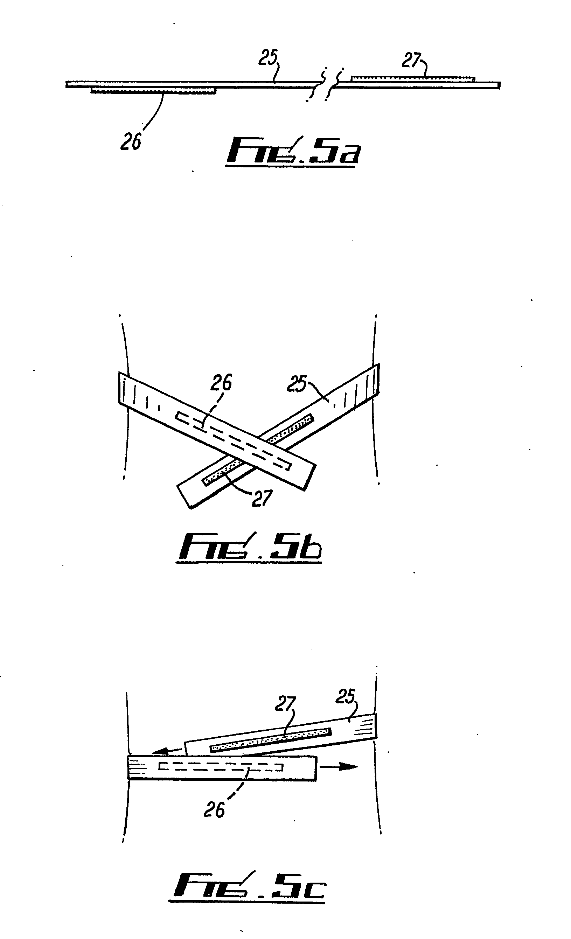

An alternative slot configuration is shown in Fig. 3, such configuration comprising a slot 21 in which the four fingers may be inserted and inner and outer holes 22, 23 for the thumb. If the crossed arm position is used to push the belt so as to tension the same, the right thumb engages the outer hole 23 of the relevant flap and the left thumb the outer hole 22 of the other flap. If a pulling action is used, the thumbs engage the inner holes of the flaps. This arrangement of holes and slots allows a superior grip on the flaps, remembering that the patient may be in a weakened condition. The crossover grip allows the patient to press the forearms upon the belly, thus increasing the support. The hole and slot arrangement allows the flaps to be securely gripped without stress upon the fingers and thumbs.

In a modification of the arrangement shown in Fig. 1, see now Fig. 4, one belt end 41 is provided with a slot 42 extending in the lengthwise direction thereof and the other belt end 43 is engaged with the said slot 42, the said other belt end 43 having a reduced width position 44 in a position spaced from the remote end thereof of a lesser dimension than the corresponding dimension of the slot. The support belt shown in Fig. 4 is used in similar manner to that of Fig. 1. It will be recognised that the belt end configuration of the Fig. 4 embodiment will avoid the need to provide the belt as two band parts arranged in overlapping end-to-end disposition, since the belt of Fig. 4 can be placed about the patient and one end then threaded through the other.

A simplified form of support belt is shown in Figs. 5a to 5c, the belt in this case being a single band 25 to extend around the patient and having a releasable fastening means 26, 27 in the overlapped end regions thereof. The band 25 is somewhat narrower than in the case of the embodiment shown in Fig. 1, and is of such width as may readily be gripped by the patient.

The releasable fastening means 26, 27 is provided merely to hold the belt loosely about the torso when not required for support purposes, the ends being separated in the use condition of the belt, and the belt being tensioned by pushing, the patient's arms being crossed, or by pulling. As can be seen in Fig. 5c, in the use condition, the belt ends will lie in adjacent side-by-side disposition, thus to provide a maximum area of support.

The invention is not limited to the exact features of the embodiments hereinbefore set forth, since alternatives will readily present themselves to one skilled in the art.

Thus, for example, whereas the tape-like elements of the embodiment shown in Figs. 1 to 3 of the drawings are formed by slitting the end regions of the bands 11, 12, equivalent structures may be provided which are secured to the respective end regions.

Whilst VELCRO (Trade Mark) type releasable fastening means are preferred, other mechanically equivalent means may be provided if desired.

## Claims

1. A post-operative support belt comprising a belt structure to encircle the patient's torso and of a length to provide overlapping free belt ends, and means to hold the belt structure loosely in endless form, as about the patient in the non-use condition thereof, the free belt ends being adapted and arranged to provide for patient-induced and patient maintained tensioning of the belt about the torso through said ends.

2. A support belt as claimed in claim 1, wherein the belt structure comprises two band parts and separable fastening means connecting said band parts in overlapping end-to-end disposition.

3. A support belt as claimed in claim 1 or 2, wherein the respective free belt ends include spaced side-by-side tape-like elements, the tape-like elements of the said ends being arranged in interdigitated disposition.

4. A support belt as claimed in claim 1 or 2, wherein one of the free belt ends includes an elongate aperture therein to receive the other free belt end therethrough.

5. A support belt as claimed in any one of the preceding claims, wherein each free belt end terminates in a respective apertured flap.

6. A support belt as claimed in claim 5, wherein the apertured flap includes an elongate aperture to receive the fingers of a users hand.

7. A support belt as claimed in claim 6, wherein the elongate aperture extends transversely of the band.

8. A support belt as claimed in claim 6, wherein the elongate aperture extends in the longitudinal direction of the band and the flap includes a further aperture arranged in transversely spaced disposition relative to the elongate aperture to receive the thumb of a users hand.

9. A support belt as claimed in claim 8, wherein a second further aperture is provided in spaced side-by-side disposition relative to the first further aperture, the first and second further apertures being approximately in transverse alignment with a respective end of the elongated aperture.

-11-

-16-

13

17

17

15

14

15

17

**FIG 1**

41

42

43

**FIG. 4**

**FIG.2**

**FIG.3**

25 27

26

**FIG.5a**

26 25

27

**FIG.5b**

27 25

26

**FIG.5c**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 641 642 (WILLIAMS, Jr.) * Abstract; column 4, lines 8-12; column 4, lines 38-56; figures * | 1,5,7 | A 61 F 5/03 |
| Y | | 3,4 | |
| Y | US-A-4 042 977 (ANTONIOUS) * Abstract; figures 22,23; column 5, lines 24-39 * | 3 | |
| Y | US-A-2 815 752 (FORMAN) * Column 1, lines 51-59; figures 1-3,9 * | 4 | |
| A | US-A-4 768 499 (KEMP) | | |

TECHNICAL FIELDS SEARCHED (Int. Cl.5)

A 61 F
A 41 F

The present search report has been drawn up for all claims

| Place of search | Date of completion of the search | Examiner |
|---|---|---|
| THE HAGUE | 25-01-1990 | SANCHEZ Y SANCHEZ J. |